# EUROPEAN PATENT SPECIFICATION

(11) **EP 3 180 091 B1**
(45) Date of publication and mention of the grant of the patent: **24.06.2020**
(21) Application number: 15753587.3
(22) Date of filing: 13.08.2015
(51) Int. Cl.: A61Q 11/00, A61K 8/21, A61K 8/22

(54) **ORAL CARE COMPOSITIONS WITH AN ENHANCED SENSORY EXPERIENCE**
MUNDPFLEGEZUSAMMENSETZUNGEN MIT VERBESSERTER SINNESERFAHRUNG
COMPOSITIONS POUR L'HYGIÈNE BUCCO-DENTAIRE AMÉLIORANT LES PERCEPTIONS SENSORIELLES

(30) Priority: 15.08.2014 US 201462038187 P
(43) Date of publication of application: 21.06.2017
(73) Proprietor: The Procter & Gamble Company, Cincinnati, OH 45202 (US)
(72) Inventor: SAGEL, Paul, Albert, Cincinnati, Ohio 45202 (US)
(74) Representative: Joos, Uta Susanne
(86) International application number: PCT/US2015/045024
(87) International publication number: WO 2016/025694

(56) References cited:
- WO-A1-00/32160
- WO-A1-00/62749
- WO-A2-2007/064885
- US-A1- 2007 237 726

## Description

### FIELD OF THE INVENTION

The present invention is directed towards a kit for an oral care regimen, particularly an oral care regimen that provides a long-lasting clean feeling.

### BACKGROUND OF THE INVENTION

People are always looking for ways to improve their smile and the health of their teeth and oral cavity. The first step on this journey for many is the use of oral care compositions like toothpastes, gels, and/or rinses. These oral care compositions can provide benefits that cover a broad range from cosmetic benefits, like reduction in staining, whitening, and fresh breath, to health benefits, like reduced tartar and antibacterial properties.

Unfortunately, components that provide the desirable health benefits, such as zinc salts, stannous salts, potassium salts, and/or copper salts (WO2007/064885), can negatively impact the overall taste and user experience. Consumers often complain that oral care products taste astringent and the taste of food and beverages is negatively impacted for an extended period of time after use.

There is a need for a kit for an oral care regimen, that can provide the desirable health benefits and cosmetic benefits, while providing sensory benefits that leave a user's mouth feeling cool, clean, and refreshed.

### SUMMARY OF THE INVENTION

A kit comprising: (a) a first dispenser containing a first oral care composition comprising stannous fluoride wherein the first composition is a dentifrice and is free of linear polyphosphates wherein the first composition has a pH from 4 to 6; (b) a second dispenser containing a second oral care composition comprising from 0.1% to 6% hydrogen peroxide wherein the second composition is a gel and does not contain any fluoride salt or an abrasive wherein the second composition has a pH from 3.5 to 5.5 and wherein the pH of the first composition is higher than the pH of the second composition; and (c) instructions wherein a user is instructed to expectorate but not rinse between applying the first composition and the second composition and wherein the instructions instruct the user to use the first composition and the second composition concurrently.

The kit as disclosed herein for reduction in staining, for whitening, for freshening breath, as antibacterial, wherein the first oral care composition is applied to an oral cavity for 30 seconds to 2 minutes; the first composition is expectorated from the oral cavity; the second oral care composition is applied for 30 seconds to 2 minutes; and wherein the first oral care composition and the second oral care composition are applied with a toothbrush and wherein the oral cavity is not rinsed between applying the first and second oral care compositions.

### BRIEF DESCRIPTION OF THE DRAWINGS

While the specification concludes with claims particularly pointing out and distinctly claiming the subject matter of the present invention, it is believed that the invention can be more readily understood from the following description taken in connection with the accompanying drawings, in which:
FIG. 1 shows the Descriptive Profile Panel (DPP) cooling intensity of the two-step regimen as compared to Colgate® Cavity Protection toothpaste; and
FIG. 2 shows the Descriptive Profile Panel (DPP) overall clean mouthfeel of the two-step regimen as compared to Colgate® Cavity Protection toothpaste.

### DETAILED DESCRIPTION OF THE INVENTION

Components that are commonly found in oral care compositions, such as zinc salts, stannous salts, potassium salts, and/or copper salts, can provide desirable health benefits but can also negatively impact the overall taste and the user's sensory experience. Consumers find that many oral care products are too astringent and the taste of foods and beverages are negatively impacted for an extended time after use.

It has been found that a two-step oral care regimen where a user brushes with a first oral care composition containing stannous fluoride, then expectorates but does not rinse, and then immediately brushes with a second oral care composition that contains hydrogen peroxide can provide the health and cosmetic benefits that users desire, as well as provide a unique sensory experience.

In one example, the first oral care composition can provide health benefits including antibacterial benefits and can contain an abrasive, such as silica, and stannous fluoride. The second oral care composition can provide cosmetic benefits including stain control, whitening and/or breath freshening benefits and can contain peroxide, namely hydrogen peroxide, wherein the second composition is an oral care gel and does not contain a fluoride salt or an abrasive.

In one example, a user can dispense the first composition onto a toothbrush and can proceed to apply the first composition to the oral cavity as part of a brushing regimen for a predetermined period of time. In one example, the first composition can be used for about one minute. In one example, the first composition can foam during use. After use of the first composition, the user can optionally expectorate the first composition. However, in one example, the user should not rinse. Then, the user sequentially dispenses the second component onto the toothbrush and applies the second component to the oral cavity as part of the brushing regimen for a predetermined amount of time. In one example, the second composition can be used for about one minute. In another example, the second composition does not foam. Then, the user can expectorate and can rinse her mouth with water. In one example, the second composition can be applied to a toothbrush or the oral cavity within 5, 10, 15, 30, 45, 60, 120, 180, 240, 300, 360, 420, seconds or 10, 15, or 20 minutes of the first component being applied to a toothbrush or the oral cavity or expectorated from the oral cavity.

The oral care compositions of the present invention not only provide health and cosmetic benefits, but the compositions can also provide a unique sensory experience for the user. This unique sensory experience can be enhanced when the products are applied sequentially, where a composition containing stannous fluoride, is applied first, and then a composition containing a hydrogen peroxide is applied second and when the user does not rinse her mouth between steps. In particular, while using the first oral care composition many users describe the first composition as warm and clean feeling in the mouth, however, consumers can also find the first composition astringent and dry. Consumers commented that when just the first oral care composition is used, the regimen can feel unfinished. However, the second composition, which contains peroxide, can provide a cool, smooth, lasting taste, that can be enjoyable to the user. Consumers can find that their mouth feels fresh and clean all day and many consumers believe that this feeling persists even after they eat and drink. After brushing some consumers described that their teeth and mouth felt glossy, shiny, vibrant, radiant, and smooth.

Another benefit is that after use a user can feel that her mouth is cool, fresh, and clean, however this feeling may not significantly interfere with the taste of food and beverages throughout the day. Users may also notice a substantial reduction in plaque on her teeth over the course of the day and even when they wake up in the morning.

However, if a user rinses her mouth between application of the first composition and the second composition, or otherwise alters the regimen, the same unique sensory experience may not be provided. For instance, the second composition may feel harsh due to the peroxide and the first composition may taste overly astringent and the flavor of food and beverages may be negatively impacted.

All percentages and ratios used hereinafter are by weight of total composition, unless otherwise indicated. All percentages, ratios, and levels of ingredients referred to herein are based on the actual amount of the ingredient, and do not include solvents, fillers, or other materials with which the ingredient may be combined as a commercially available product, unless otherwise indicated.

All measurements referred to herein are made at 25°C (i.e. room temperature) unless otherwise specified.

The composition can contain, consist of, or consist essentially of, the essential elements and limitations of the invention described herein, as well as any additional or optional ingredients, components, or limitations described herein or otherwise useful in oral care compositions.

As used herein, the word "include," and its variants, are intended to be non-limiting, such that recitation of items in a list is not to the exclusion of other like items that may also be useful in the materials, compositions, devices, and methods of this invention.

As used herein, the word "or" when used as a connector of two or more elements is meant to include the elements individually and in combination; for example X or Y, means X or Y or both.

By "oral care composition", as used herein, is meant a product, which in the ordinary course of usage, is not intentionally swallowed for purposes of systemic administration of particular therapeutic agents, but is rather retained in the oral cavity for a time sufficient to contact dental surfaces or oral tissues. Examples of oral care compositions include dentifrice, mouth rinse, mousse, foam, mouth spray, lozenge, chewable tablet, chewing gum, tooth whitening strips, floss and floss coatings, breath freshening dissolvable strips, or denture care or adhesive product. The oral care composition may also be incorporated onto strips or films for direct application or attachment to oral surfaces.

The term "dentifrice", as used herein, includes tooth or subgingival -paste, gel, or liquid formulations unless otherwise specified. The dentifrice composition may be a single phase composition or may be a combination of two or more separate dentifrice compositions. The dentifrice composition may be in any desired form, such as deep striped, surface striped, multilayered, having a gel surrounding a paste, or any combination thereof. Each dentifrice composition in a dentifrice comprising two or more separate dentifrice compositions may be contained in a physically separated compartment of a dispenser and dispensed side-by-side.

The term "dispenser", as used herein, means any pump, tube, or container suitable for dispensing compositions such as dentifrices.

The term "teeth", as used herein, refers to natural teeth as well as artificial teeth or dental prosthesis.

The two-step oral care regimen can include a first oral care composition that can contain a fluoride salt and a second oral care composition that can contain hydrogen peroxide. If a user brushes with the first composition, then expectorates the first composition but does not rinse, and then brushes with the second composition, the user can have a unique sensory experience, in addition to health and cosmetic benefits.

Sixteen consumers used the two-step regimen, as described in the example herein, for two weeks. The consumers generally described the first composition as having a warm and clean feeling in the mouth, however, some consumers found the first composition astringent and drying. Some consumers indicated that the first step felt like their teeth were being stripped of all grime, which includes plaque and food that can build up on the teeth between brushes. Before applying the second composition, many consumers thought that their brushing felt unfinished. However, when they used the second composition, which contained peroxide and a different and complimentary flavor to the first composition, many consumers indicated that their mouth felt cool and smooth. Consumers generally agreed that the two-step regimen provided a long-lasting clean feeling that persisted throughout the day without the hassle of having to use gums, rinse, or brush their teeth during the day, especially after meals or drinking. Many consumers indicated that they felt the two-step regimen provided a deep clean, especially when compared to their current dental hygiene regimen.

Twelve of the sixteen consumers self-identified as lovers of the regimen and the most commonly communicated benefits from these consumers are as follows:
- An unprecedented, squeaky, all-day clean
- A clean I can feel and see
- I can see and feel the clean even after eating, drinking, and at the end of a long day.
- I noticed, but others noticed as well.
- I believe/ know my dentist will notice.
- I can see the two steps working together.

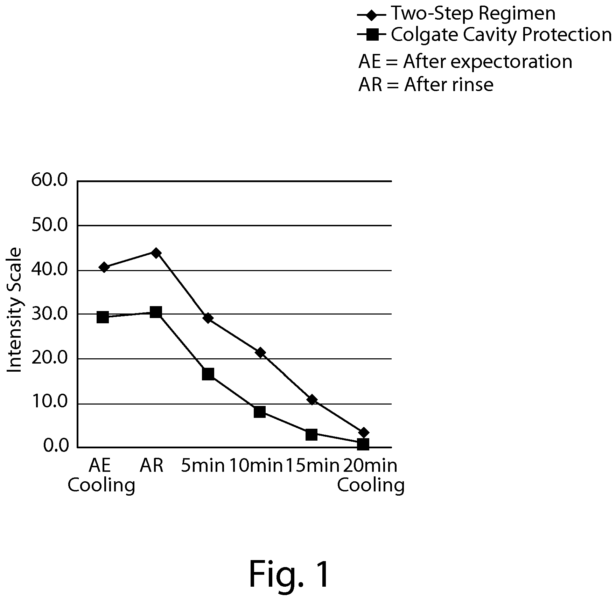

Furthermore, the two-step regimen can provide an exceptionally cool feeling in a user's mouth, especially after expectoration and rinsing of the second composition. FIG. 1 shows the cooling intensity, as determined by the DPP panel, vs. Time for the two-step regimen, as described in the examples hereafter, and Colgate® Cavity Protection toothpaste.

The DPP panel was conducted as follows, on the morning of the sample evaluations, the panel was instructed not to brush or use any oral care and/or breath refreshing products. Each panelist brushed with the two-step regimen or the Colgate® Cavity Protection toothpaste (Lot # MBT0732-016). For the two-step process the panelists brushed with the first composition for one minute, expectorated the first composition but did not rinse, brushed with the second composition for one minute, and then expectorated and rinsed. For the Colgate® Cavity Protection toothpaste the panelists brushed for two minutes, expectorated, and then rinsed. The DPP panel includes panelists that are trained and validated in Spectrum™ Descriptive Analysis methodology and evaluate cooling on a 60 point scale, with 60 being the most cooling.

It was found that the two-step regimen had significantly more cooling after the second composition was expectorated and after the second composition was rinsed, as compared to Colgate® Cavity Protection. This difference was large enough that it would be noticeable by consumers. The cooling effect was also greater than Colgate® Cavity Protection for 10 minutes and 15 minutes after expectoration than the Colgate® Cavity Protection and this difference may be consumer noticeable.

In one example, the oral care composition can have a cooling of greater than about 30 after expectoration as determined by the DPP panel, in another example greater than about 33, in another example greater than about 35, in another example greater than about 37, and in another example greater than about 40. In another example, the oral care composition can have a cooling of greater than about 33 after expectoration as determined by the DPP panel, in another example greater than about 35, in another example greater than about 38, in another example greater than about 40, and in another example greater than about 42. In yet another example, the oral care composition can have a cooling of greater than about 20 five minutes after expectoration as determined by the DPP panel, in another example greater than about 25, and in another example greater than about 28.

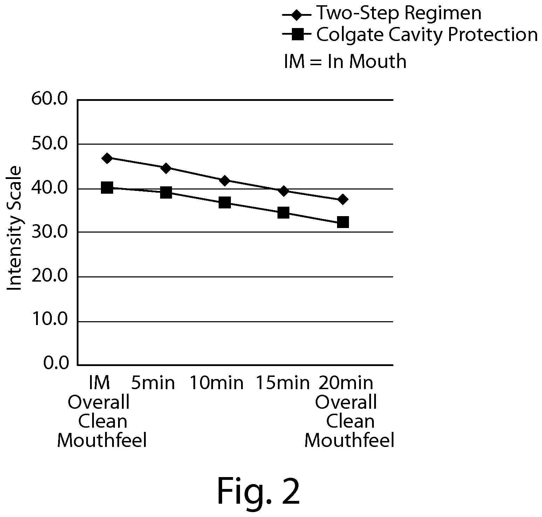

Furthermore, the two-step regimen can provide an overall clean mouthfeel. FIG. 2 shows the overall clean mouthfeel, as determined by the DPP panel, vs. Time for the two-step regimen, as described in the examples hereafter, and Colgate® Cavity Protection toothpaste. The DPP panel followed the same procedure as described above and evaluated clean mouthfeel on a 60 point scale, with 60 being the most clean.

As shown in FIG. 2, the two-step regimen rated higher for overall clean mouthfeel than Colgate® Cavity Protection at all time points that were measured, including in mouth, 5 minutes, 10 minutes, 15 minutes, and 20 minutes after expectoration.

In one example, the two-step regimen can have an overall clean mouthfeel of greater than about 30 twenty minutes after expectoration as determined by the DPP panel, in another example greater than about 33, in another example greater than about 35, and in another example greater than about 37.

Furthermore, the two-step regimen can reduce bad breath for an extended period of time. In one example, before brushing the mean VSCs (volatile sulfur compounds) for 29 subjects was greater than about 150. After brushing with the two-step regimen and then waiting 24 hours, the mean VSCs was less than about 120. The human detection for being able to smell bad breath is greater than about 120 and thus, even after 24 hours, bad breath was undetectable or almost undetectable when the two-step regimen was used.

In some examples, the two-step regimen does not interfere with the taste of foods and beverages for a significant period of time. In one example, the two-step regimen stops interfering with the taste of foods and beverages after at least 20 minutes have passed, in another example after at least 30 minutes have passed, in another example after at least 45 minutes have passed, in another example after at least 60 minutes have passed, and in another example after at least 90 minutes have passed.

A wide variety of oral care devices can be used to dispense the oral care compositions, including manual toothbrush, electric toothbrushes, and a variety of other packages (e.g. hand pumps, etc.) and devices. In one example, the toothbrush can be a manual brush with extra soft, soft, medium, and/or firm bristles. In one example, a user may start a regimen with a brush with extra soft bristles or soft bristles and then can transition to firmer bristles as she gets used to the regimen and/or her oral hygiene improves. In one example, a user can use both a power brush to apply the first oral care composition and then a manual brush to apply the second oral care composition. In one example, the first composition, the second composition, and a manual toothbrush are packaged together in a kit. In another example, the user can be instructed not to use an electric toothbrush.

In one example, a user can brush with the first or second composition for 15 seconds to 2.5 minutes, in another example from 30 seconds to 2 minutes, in another example from 45 seconds to 90 seconds, and in another example from 1 minute to 75 seconds. In another example, a user can brush with the first component for 1 to 2 minutes and then the second component for 15 seconds to 1 minute. In another example, a user can use the first component for longer than the second component. In another example, the user can use the first component for approximately the same time as the second component. In another example, the user can use the first component for less time than the second component.

In one example, the first composition can be administered after the second composition. In another example, the first composition and the second composition can be stored separately but can be administered concurrently.

In one example, the first oral care composition can be a dentifrice composition and the second oral care composition can be a rinse. In one example, the rinse can contain hydrogen peroxide.

In one example, the first composition and the second composition can contain different flavors to enhance the sensory signal to the user. In one example, the flavor change may indicate that the user should brush longer or can terminate brushing.

In one example, a user can use the first composition and the second composition as needed or two times a day. In another example, a user can use the first composition as needed or two times a day and the second composition can be used once a day or in another example the second composition can be used every other day, or once a week. In another example, a user can use the first composition as needed or two times a day and the second composition is only used when the user wants enhanced cosmetic benefits, such as whitening.

The first and second oral care compositions can be provided in completely separate packages, which may be bundled together as a kit. For example, a first composition provided as a dentifrice could be provided in a first dentifrice dispenser and a second component provided as a gel could be provided in a second separate dispenser. The first and second dispensers may be the same or different. The first and second packages could be provided in visually distinct shapes, sizes, or color(s) so that a user can easily differentiate between the two. The first and second packages might also be provided with graphics, text, icons, or numeric characters to differentiate between the two. In some examples, the first and second packages can meter the first and second compositions so that a particular dose can be delivered resulting in application of a controlled ratio between the first and second components. In another example, the first package and second package are oriented in different directions, in one example the first package has an upright orientation and the second package has a downward orientation. In another example, the first package and the second package have the same orientation. In another example, the first package and the second package are packaged together in a secondary container. The secondary container can have at least a portion of one or more panels that can be transparent, which can allow a user to see all or a portion of the first and/or second package. In one example, a front panel of the secondary package can be transparent. In another example, the kit can include a first package containing a first oral care composition, a second package containing a second oral care composition, and one or more manual toothbrushes where at least one toothbrush has soft or extra soft bristles.

In one example, the weight ratio of first composition to second composition can be from 0.5:1 to 3:1, in another example from 0.75:1 to 2:1, and in another example from 1:1 to 1.5:1. In another example, the weight ratio of first composition to second composition can be 1:1.

In one example, the specific gravity of the first composition can be greater than the specific gravity of the second composition. In one example the specific gravity of the first composition can be from 0.5 to 2.5, in another example from 0.75 to 2, in another example from 1 to 1.75, and in another example from 1.25 to 1.50. In another example the specific gravity of the second composition can be from 0.25 to 2, in another example from 0.5 to 1.5, in another example from 0.75 to 1.25, and in another example from 1.0 to 1.15. In another example, the ratio of the specific gravity of the first composition to the specific gravity of the second composition can be from 0.5:2 to 3:1, in another example from 0.75:1.5 to 2:1, in another example from 1:1 to 1.5:1, and in another example from 1.1:1 to 1.3:1.

In one example, the viscosity of the first composition can be greater than the viscosity of the second composition. In one example, the viscosity of the first composition can be from 5 to 40 BKUs. In another example, the viscosity of the second composition can be from 5 to 25 BKUs. The viscosity is measured with a Brookfield Synchrolectric Viscometer Model RVT/2 using a T-E spindle at 2.5 revolutions per minute.

The first composition is and the second composition can be free of a polyphosphate. In another example, the first and/or the second composition can be substantially free of linear "glassy" polyphosphates having the having the formula:

XO(XPO₃)ₙX

wherein X is sodium, potassium or ammonium and n averages from about 6 to about 125.

The second composition is free from abrasives. In another example, the second composition can be substantially free of solids.The second composition is a gel.

In another example, the first composition can contain less water than the second composition. In one example the first composition can contain from 15% to 50% water, in another example from 20% to 40% water, and in another example from 25% to 30% water. In another example, the second composition can contain from 50% to 90% water, in another example from 60% to 80%, and in another example from 65% to 75% water.

The first composition has a higher pH than the second composition. The first composition has a pH from 4 to 6, and in another example from 5.25 to 6.25. The second composition has a pH from 3.5 to 5.5, and in another example from 4.75 to 5.25. The pH can be determined by the pH Test Method as described hereafter.

The second oral care composition contains from 0.1% to 6% hydrogen peroxide, in another example from 0.2% to 6% hydrogen peroxide, in another example from 0.3% to 5% hydrogen peroxide, in another example from 0.5% to 4% hydrogen peroxide, in another example from 0.75% to 3.75% hydrogen peroxide, in another example from 1% to 3.5% hydrogen peroxide, in another example from 1.5% to 3.3% hydrogen peroxide, in another example from 2% to 3.25% hydrogen peroxide, in another example from 2.5% to 3.2%, and in another example from 2.75% to 3.1% hydrogen peroxide. In another example, the composition comprises from greater than 0.3% hydrogen peroxide, in another example greater than 0.5% hydrogen peroxide, in another example greater than 1% hydrogen peroxide, in another example greater than 2% hydrogen peroxide, in another example greater than 2.5% hydrogen peroxide, in another example greater than 2.8% hydrogen peroxide, in another example greater than 2.9% hydrogen peroxide, and in another example greater than 3% hydrogen peroxide.

Actives and other ingredients may be categorized or described herein by their cosmetic benefit, therapeutic benefit, or their postulated mode of action or function. However, it is to be understood that the active and other ingredients useful herein can, in some instances, provide more than one cosmetic benefit, therapeutic benefit, function, or can operate via more than one mode of action. Therefore, classifications herein are made for the sake of convenience and are not intended to limit an ingredient to the particularly stated function(s) or activities listed.

A metal salt includes zinc salts, stannous salts, potassium salts, copper salts, alkali metal bicarbonate slats, and combinations thereof. Metal salts have a wide range of functions from antimicrobial agents to sensitivity agents or buffers. The oral care compositions of the present invention may contain metal salt in an amount from about 0.05% to about 11%, from about 0.5% to about 7%, or from about 1% to about 5%, by total weight of the oral care composition.

It is common to have a fluoride compound present in dentifrices and other oral care compositions in an amount sufficient to give a fluoride ion concentration in the composition of from about 0.0025% to about 5.0% or from about 0.005% to about 2.0%, by weight of the oral care composition to provide anticaries effectiveness. A wide variety of fluoride ion-yielding materials can be employed as sources of soluble fluoride in the present invention. Representative fluoride ion sources include: stannous fluoride, sodium fluoride, potassium fluoride, amine fluoride, sodium monofluorophosphate, indium fluoride, amine fluorides such as Olaflur, and many others. Examples of suitable fluoride ion-yielding materials are found in U.S. Pat. No. 3,535,421 to Briner et al. and U.S. Pat. No. 3,678,154 to Widder et al.

Stannous salts include stannous fluoride, stannous chloride, stannous iodide, stannous chlorofluoride, stannous actetate, stannous hexafluorozirconate, stannous sulfate, stannous lactate, stannous tartrate, stannous gluconate, stannous citrate, stannous malate, stannous glycinate, stannous pyrophosphate, stannous metaphosphate, stannous oxalate, stannous phosphate, stannous carbonate, and combinations thereof. Dentifrices containing stannous salts, particularly stannous fluoride and stannous chloride, are described in U.S. Pat. No. 5,004,597 to Majeti et al. Other descriptions of stannous salts are found in U.S. Pat. No. 5,578,293 issued to Prencipe et al. and in U.S. Pat. No. 5,281,410 issued to Lukacovic et al. In addition to the stannous ion source, other ingredients used to stabilize the stannous may be included, such as the ingredients described in Majeti et al. and Prencipe et al.

Zinc salts include zinc fluoride, zinc chloride, zinc iodide, zinc chlorofluoride, zinc actetate, zinc hexafluorozirconate, zinc sulfate, zinc lactate, zinc tartrate, zinc gluconate, zinc citrate, zinc malate, zinc glycinate, zinc pyrophosphate, zinc metaphosphate, zinc oxalate, zinc phosphate, zinc carbonate, and combinations thereof.

Potassium salts include potassium nitrate, potassium citrate, potassium oxalate, potassium bicarbonate, potassium acetate, potassium chloride, and combinations thereof.

In one example, the copper salt can be selected from copper fluoride, copper chloride, copper iodide, copper chlorofluoride, copper actetate, copper hexafluorozirconate, copper sulfate, copper lactate, copper tartrate, copper gluconate, copper citrate, copper malate, copper glycinate, copper pyrophosphate, copper metaphosphate, copper oxalate, copper phosphate, copper carbonate, and combinations thereof. In a further example, the copper salt can be selected from copper gluconate, copper acetate, copper glycinate, and combinations thereof.

Alkali metal bicarbonate salts are soluble in water and unless stabilized, tend to release carbon dioxide in an aqueous system. Sodium bicarbonate, also known as baking soda, can be the preferred alkali metal bicarbonate salt. The alkali metal bicarbonate salt also functions as a buffering agent. Because of the pH at which alkali metal bicarbonate salts buffer, the bicarbonate salt may be in a phase separate from the stannous ion source. In certain examples, the oral care composition of the present invention may contain from about 0.5% to about 50%, from about 0.5% to about 30%, from about 2% to about 20%, or from about 5% to about 18% of an alkali metal bicarbonate salt, by weight of the oral care composition.

Some metal salts which may be used in the present invention, such as zinc chloride, zinc citrate, copper gluconate, and zinc gluconate, are also associated with an off taste described as dirty, dry, earthy, metallic, sour, bitter, and astringent.

Sweeteners include saccharin, chloro-sucrose (sucralose), steviolglycosides, rebaudioside A, rebaudioside B, rebaudioside C, rebaudioside D, rebaudioside E, rebaudioside F, dulcoside A, dulcoside B, rubusoside, stevia, stevioside, acesulfame K, xylitol, neohesperidine DC, alitame, aspartame, neotame, alitame, thaumatin, cyclamate, glycyrrhizin, mogroside IV, mogroside V, Luo Han Guo sweetener, siamenoside, monatin and its salts (monatin SS, RR, RS, SR), curculin, monellin, mabinlin, brazzein, hemandulcin, phyllodulcin, glycyphyllin, phloridzin, trilobatin, baiyanoside, osladin, polypodoside A, pterocaryoside A, pterocaryoside B, mukurozioside, phlomisoside I, periandrin I, abrusoside A, cyclocarioside I,N-[N-[3-(3-hydroxy-4-methoxyphenyl)propyl]-L-a-aspartyl]-L-phenylalanine 1-methyl ester, N-[N-[3-(3-hydroxy-4-methoxyphenyl)-3-methylbutyl]-L-α-aspartyl]-L-phenylalanine 1-methyl ester, N-[N-[3-(3-methoxy-4-hydroxyphenyl)propyl]-L-α-aspartyl]-L-phenylalanine 1-methyl ester, salts thereof, and combinations thereof.

Rebiana can be a steviolglycoside from Cargill Corp., Minneapolis, MN, which is an extract from the leaves of the Stevia rebaudiana plant (hereinafter referred to as "Rebiana"). This is a crystalline diterpene glycoside, about 300x sweeter than sucrose. Examples of suitable stevioglycosides which may be combined include rebaudioside A, rebaudioside B, rebaudioside C, rebaudioside D, rebaudioside E, rebaudioside F, dulcoside A, dulcoside B, rubusoside, stevioside, or steviolbioside. According to particularly desirable examples of the present invention, the combination of high-potency sweeteners comprises rebaudioside A in combination with rebaudioside B, rebaudioside C, rebaudioside F, rebaudioside F, stevioside, steviolbioside, dulcoside A. Sweeteners are generally included in an oral care composition at a level of about 0.0005% to about 2 %, by total weight of the oral care composition.

Carrier materials include water, glycerin, sorbitol, polyethylene glycols having a molecular weight of less than about 50,000, propylene glycol and other edible polyhydric alcohols, ethanol, or combinations thereof. The oral care compositions of the present invention include from about 5% to about 80%, by weight of the composition, of a carrier material. In certain examples, the compositions contain carrier materials in an amount of from about 10% to about 40%, by total weight of the oral care composition.

Antimicrobial agents include quaternary ammonium compounds. Those useful in the present invention include, for example, those in which one or two of the substitutes on the quaternary nitrogen has a carbon chain length (typically alkyl group) from about 8 to about 20, typically from about 10 to about 18 carbon atoms while the remaining substitutes (typically alkyl or benzyl group) have a lower number of carbon atoms, such as from about 1 to about 7 carbon atoms, typically methyl or ethyl groups. Dodecyl trimethyl ammonium bromide, tetradecylpyridinium chloride, domiphen bromide, N-tetradecyl-4-ethyl pyridinium chloride, dodecyl dimethyl (2-phenoxyethyl) ammonium bromide, benzyl dimethoylstearyl ammonium chloride, quaternized 5-amino-1,3-bis(2-ethyl-hexyl)-5-methyl hexahydropyrimidine, benzalkonium chloride, benzethonium chloride and methyl benzethonium chloride are exemplary of typical quaternary ammonium antibacterial agents.

Other quaternary ammonium compounds include the pyridinium compounds. Examples of pyridinium quaternary ammonium compounds include bis[4-(R-amino)-1-pyridinium] alkanes as disclosed in U.S. Pat. No. 4,206,215, Jun. 3, 1980, to Bailey and cetylpyridinium and tetradecylpyridinium halide salts (i.e., chloride, bromide, fluoride and iodide).

The oral care compositions of the present invention may also include other antimicrobial agents including non-cationic antimicrobial agents such as halogenated diphenyl ethers, phenolic compounds including phenol and its homologs, mono and poly-alkyl and aromatic halophenols, resorcinol and its derivatives, xylitol, bisphenolic compounds and halogenated salicylanilides, benzoic esters, and halogenated carbanilides. Also useful antimicrobials are enzymes, including endoglycosidase, papain, dextranase, mutanase, and combinations thereof. Such agents are disclosed in U.S. Pat. No. 2,946,725, Jul. 26, 1960, to Norris et al. and in U.S. Pat. No. 4,051,234 to Gieske et al. Examples of other antimicrobial agents include chlorhexidine, and flavor oils such as thymol. In another example, the antimicrobial agent can include triclosan.

The compositions of the present invention may contain antimicrobial agents in an amount of from about 0.035% or more, from about 0.1% to about 1.5%, from about 0.045% to about 1.0%, or from about 0.05% to about 0.10%, by total weight of the oral care composition.

Bleaching agents can include peroxides, perborates, percarbonates, peroxyacids, persulfates, and combinations thereof. Suitable peroxide compounds include hydrogen peroxide, urea peroxide, calcium peroxide, sodium peroxide, zinc peroxide, or combinations thereof. One example of a percarbonate is sodium percarbonate. An example of a persulfate includes oxones. Some bleaching agents provide a burn sensation within an oral care composition, for example peroxides and percarbonates.

The compositions of the present invention may contain bleaching agents in an amount of from about 0.01% to about 30%, from about 0.1% to about 10%, or from about 0.5% to about 5%, by total weight of the oral care composition.

Surfactants may include anionic surfactants such as organophosphate, which include alkyl phosphates. These surface active organophosphate agents have a strong affinity for enamel surface and have sufficient surface binding propensity to desorb pellicle proteins and remain affixed to enamel surfaces. Suitable examples of organophosphate compounds include mono-, di- or triesters represented by the general structure below wherein Z1, Z2, or Z3 may be identical or different, at least one being an organic moiety, in one example selected from linear or branched, alkyl or alkenyl group of from 1 to 22 carbon atoms, optionally substituted by one or more phosphate groups; alkoxylated alkyl or alkenyl, (poly)saccharide, polyol or polyether group.

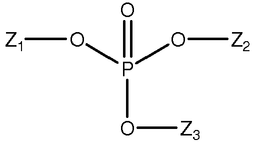

Some other organophosphate agents include alkyl or alkenyl phosphate esters represented by the following structure: 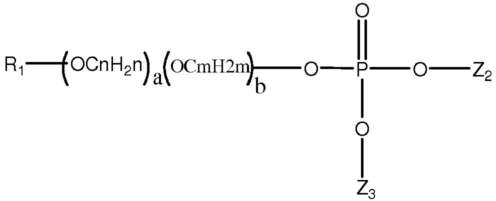 wherein R1 represents a linear or branched, alkyl or alkenyl group of from 6 to 22 carbon atoms, optionally substituted by one or more phosphate groups; n and m, are individually and separately, 2 to 4, and a and b, individually and separately, are 0 to 20; Z2 and Z3 may be identical or different, each represents hydrogen, alkali metal, ammonium, protonated alkyl amine or protonated functional alkyl amine such as an alkanolamine, or a R1-(OCnH2n)a(OCmH2m)b-group. Examples of suitable agents include alkyl and alkyl (poly)alkoxy phosphates such as lauryl phosphate; PPG5 ceteareth-10 phosphate; Laureth-1 phosphate; Laureth-3 phosphate; Laureth-9 phosphate; Trilaureth-4 phosphate; C12-18 PEG 9 phosphate; Sodium dilaureth-10 phosphate. In one example, the alkyl phosphate is polymeric. Examples of polymeric alkyl phosphates include those containing repeating alkoxy groups as the polymeric portion, in particular 3 or more ethoxy, propoxy isopropoxy or butoxy groups.

Zwitterionic or amphoteric surfactants useful in the present invention can include derivatives of aliphatic quaternary ammonium, phosphonium, and sulfonium compounds, in which the aliphatic radicals can be straight chain or branched, and wherein one of the aliphatic substituents contains from about 8 to 18 carbon atoms and one contains an anionic water-solubilizing group, such as carboxy, sulfonate, sulfate, phosphate or phosphonate. Suitable amphoteric surfactants include betaine surfactants such as disclosed in U.S. Pat. No. 5,180,577 to Polefka et al. Typical alkyl dimethyl betaines include decyl betaine or 2-(N-decyl-N,N-dimethylammonio) acetate, coco betaine or 2-(N-coco-N, N-dimethyl ammonio) acetate, myristyl betaine, palmityl betaine, lauryl betaine, cetyl betaine, stearyl betaine, etc. Amphoteric surfactants useful herein further include amine oxide surfactants. The amidobetaines are exemplified by cocoamidoethyl betaine, cocamidopropyl betaine (CAPB), and lauramidopropyl betaine. The unwanted tastes often associated with these surfactants are soapy, bitter, chemical, or artificial.

Additional suitable polymeric organophosphate agents can include dextran phosphate, polyglucoside phosphate, alkyl polyglucoside phosphate, polyglyceryl phosphate, alkyl polyglyceryl phosphate, polyether phosphates and alkoxylated polyol phosphates. Some specific examples are PEG phosphate, PPG phosphate, alkyl PPG phosphate, PEG/PPG phosphate, alkyl PEG/PPG phosphate, PEG/PPG/PEG phosphate, dipropylene glycol phosphate, PEG glyceryl phosphate, PBG (polybutylene glycol) phosphate, PEG cyclodextrin phosphate, PEG sorbitan phosphate, PEG alkyl sorbitan phosphate, and PEG methyl glucoside phosphate. Suitable non-polymeric phosphates include alkyl mono glyceride phosphate, alkyl sorbitan phosphate, alkyl methyl glucoside phosphate, alkyl sucrose phosphates. The impurities in these phosphates may induce a burning sensation. Impurities may include dodecanol, dodecanal, benzaldehyde, and other TRPA1 or TRPV1 agonists.

Cationic surfactants useful in the present invention can include derivatives of quaternary ammonium compounds having one long alkyl chain containing from about 8 to 18 carbon atoms such as lauryl trimethylammonium chloride, cetyl trimethylammonium bromide, coconut alkyltrimethylammonium nitrite, cetyl pyridinium fluoride, etc. Quaternary ammonium halides having detergent properties can be used, such as those described in U.S. Pat. No. 3,535,421 to Briner et al. Certain cationic surfactants can also act as germicides in the oral care compositions disclosed herein.

Examples of some flavors and flavor components that may be used in oral care compositions are mint oils, wintergreen, clove bud oil, cassia, sage, parsley oil, marjoram, lemon, orange, propenyl guaethol, heliotropine, 4-cis-heptenal, diacetyl, methyl-p-tert-butyl phenyl acetate, methyl salicylate, ethyl salicylate, 1-menthyl acetate, oxanone, α-irisone, methyl cinnamate, ethyl cinnamate, butyl cinnamate, ethyl butyrate, ethyl acetate, methyl anthranilate, iso-amyl acetate, iso-amyl butyrate, allyl caproate, eugenol, eucalyptol, thymol, cinnamic alcohol, octanol, octanal, decanol, decanal, phenylethyl alcohol, benzyl alcohol, α-terpineol, linalool, limonene, citral, neral, geranial, geraniol nerol, maltol, ethyl maltol, anethole, dihydroanethole, carvone, menthone, β-damascenone, ionone, γ-decalactone, γ-nonalactone, γ-undecalactone, or combinations thereof. Generally suitable flavoring ingredients are chemicals with structural features and functional groups that are less prone to redox reactions. These include derivatives of flavor chemicals that are saturated or contain stable aromatic rings or ester groups.

Flavors are generally present in an amount of from about 0.4 % to about 5% or from about 1% to about 3%, by total weight of the oral care composition.

Anti-tartar agents include pyrophosphate salts as a source of pyrophosphate ion. The pyrophosphate salts useful in the present compositions include, for example, the mono-, di- and tetraalkali metal pyrophosphate salts and combinations thereof. Disodium dihydrogen pyrophosphate (Na2H2P2O7), sodium acid pyrophosphate, tetrasodium pyrophosphate (Na4P2O7), and tetrapotassium pyrophosphate (K4P2O7) in their unhydrated as well as hydrated forms are further species. In compositions of the present invention, the pyrophosphate salt may be present in one of three ways: predominately dissolved, predominately undissolved, or a combination of dissolved and undissolved pyrophosphate. The amount of pyrophosphate salt useful in making these compositions is any tartar control effective amount. In varying examples, the amount of pyrophosphate salt may be from about 1.5% to about 15%, from about 2% to about 10%, or about 3% to about 8%, by total weight of the oral care composition.

Examples of some colorants that may be used in oral care compositions include D&C Yellow No. 10, FD&C Blue No. 1, FD&C Red No. 40, D&C Red No. 33 and combinations thereof. In certain examples, the composition comprises colorant in an amount of from about 0.0001 % to about 0.1% or from about 0.001% to about 0.01%, by weight of the oral care composition. Some colorants provide an unwanted taste, for example, D&C Red No. 33. The unwanted tastes often associated with this colorant are metallic, sharp, or chemical. Colorants are generally present in an amount of from about 0.001% to about 0.5%, by weight of the oral care composition.

Sensates may also be part of an oral care composition. Sensate molecules such as cooling, warming, and tingling agents are useful to deliver signals to the user. Sensates are generally present in an amount of from about 0.001% to about 0.8%, by weight of the oral care composition. The most well-known cooling sensate compound can be menthol, particularly L-menthol, which is found naturally in peppermint oil notably of Mentha arvensis L and Mentha viridis L. Other isomers of menthol (neomenthol, isomenthol and neoisomenthol) have somewhat similar, but not identical odor and taste, for instance having disagreeable odor and taste described as earthy, camphor, musty, etc. The biggest difference among the isomers is in their cooling potency. L-menthol provides the most potent cooling, by having the lowest cooling threshold of about 800 ppb, which is the concentration level where the cooling effect can be clearly recognized. At this level, there can be no cooling effect for the other isomers. For example, d-neomenthol is reported to have a cooling threshold of about 25,000 ppb and l-neomenthol about 3,000 ppb.

Of the menthol isomers the l-isomer occurs most widely in nature and is typically what is referred by the name menthol having coolant properties. L-menthol has the characteristic peppermint odor, has a clean fresh taste and exerts a cooling sensation when applied to the skin and mucosal surfaces.

Among synthetic coolants, many are derivatives of or are structurally related to menthol, for example containing the cyclohexane moiety, and derivatized with functional groups including carboxamide, ketal, ester, ether and alcohol. Examples include the p-menthanecarboxamide compounds such as N-ethyl-p-menthan-3-carboxamide, known commercially as "WS-3", and others in the series such as WS-5 (N-ethoxycarbonylmethyl-p-menthan-3-carboxamide), WS-12 (1R*,2S*)-N-(4-Methoxyphenyl)-5-methyl-2-(1-methylethyl)cyclohexanecarboxamide] and WS-14 (N-tert-butyl-p-menthan-3-carboxamide). Examples of menthane carboxy esters include WS-4 and WS-30. An example of a synthetic carboxamide coolant that is structurally unrelated to menthol is N,2,3-trimethyl-2-isopropylbutanamide, known as "WS-23". Additional examples of synthetic coolants include alcohol derivatives such as 3-(1-menthoxy)-propane-1,2-diol known as TK-10, isopulegol (under the tradename Coolact P) and p-menthane-3,8-diol (under the tradename Coolact 38D) all available from Takasago Corp., Tokyo, Japan; menthone glycerol acetal known as MGA; menthyl esters such as menthyl acetate, menthyl acetoacetate, menthyl lactate known as Frescolat® supplied by Symrise AG, Holzminden, Germany, and monomenthyl succinate under the tradename Physcool from V. Mane FILS, Notre Dame, France. TK-10 is described in U.S. Pat. No. 4,459,425 to Amano et al. Other alcohol and ether derivatives of menthol are described in GB 1,315,626 and in U.S. Pat. Nos. 4,029,759; 5,608,119; and 6,956,139. WS-3 and other carboxamide cooling agents are described in U.S. Pat. No's 4,136,163; 4,150,052; 4,153,679; 4,157,384; 4,178,459 and 4,230,688.

Additional N-substituted p-menthane carboxamides are described in WO 2005/049553A1 including N-(4-cyanomethylphenyl)-ρ-menthanecarboxamide, N-(4-sulfamoylphenyl)-ρ-menthanecarboxamide, N-(4-cyanophenyl)p-menthanecarboxamide, N-(4-acetylphenyl)-p-menthanecarboxamide, N-(4-hydroxymethylphenyl)-p-menthanecarboxamide and N-(3-hydroxy-4-methoxyphenyl)-p-menthanecarboxamide. Other N-substituted p-menthane carboxamides include amino acid derivatives such as those disclosed in WO 2006/103401 and in U.S. Pat. Nos. 4,136,163; 4,178,459 and 7,189,760 such as N-((5-methyl-2-(1-methylethyl)cyclohexyl)carbonyl)glycine ethyl ester and N-((5-methyl-2-(1-methylethyl)cyclohexyl)carbonyl)alanine ethyl ester. Menthyl esters including those of amino acids such as glycine and alanine are disclosed e.g., in EP 310,299 and in U.S. Pat. Nos. 3,917,613; 3,991,178; 5,703,123; 5,725,865; 5,843,466; 6,365,215; and 6,884,903. Ketal derivatives are described, e.g., in U.S. Pat. Nos. 5,266,592; 5,977,166; and 5,451,404. Additional agents that are structurally unrelated to menthol but have been reported to have a similar physiological cooling effect include alpha-keto enamine derivatives described in U.S. Pat. No. 6,592,884 including 3-methyl-2-(1-pyrrolidinyl)-2-cyclopenten-1-one (3-MPC), 5-methyl-2-(1-pyrrolidinyl)-2-cyclopenten-1-one (5-MPC), and 2,5-dimethyl-4-(1-pyrrolidinyl)-3(2H)-furanone (DMPF); icilin (also known as AG-3-5, chemical name 1-[2-hydroxyphenyl]-4-[2-nitrophenyl]-1,2,3,6-tetrahydropyrimidine-2-one) described in Wei et al., J. Pharm. Pharmacol. (1983), 35:110-112. Reviews on the coolant activity of menthol and synthetic coolants include H. R. Watson, et al. J. Soc. Cosmet. Chem. (1978), 29, 185-200 and R. Eccles, J. Pharm. Pharmacol., (1994), 46, 618-630.

Additional agents that are structurally unrelated to menthol but have been reported to have a similar physiological cooling effect include alpha-keto enamine derivatives described in U.S. Pat. No. 6,592,884 including 3-methyl-2-(1-pyrrolidinyl)-2-cyclopenten-1-one (3-MPC), 5-methyl-2-(1-pyrrolidinyl)-2-cyclopenten-1-one (5-MPC), and 2,5-dimethyl-4-(1-pyrrolidinyl)-3(2H)-furanone (DMPF); icilin (also known as AG-3-5, chemical name 1-[2-hydroxyphenyl]-4-[2-nitrophenyl]-1,2,3,6-tetrahydropyrimidine-2-one) described in Wei et al., J. Pharm. Pharmacol. (1983), 35:110-112 and phosphine oxides as reported in U.S. Pat. No. 4,070,496.

Some examples of warming sensates include ethanol; capsicum; nicotinate esters, such as benzyl nicotinate; polyhydric alcohols; capsicum powder; a capsicum tincture; capsicum extract; capsaicin; homocapsaicin; homodihydrocapsaicin; nonanoyl vanillyl amide; nonanoic acid vanillyl ether; vanillyl alcohol alkyl ether derivatives such as vanillyl ethyl ether, vanillyl butyl ether, vanillyl pentyl ether, and vanillyl hexyl ether; isovanillyl alcohol alkyl ethers; ethylvanillyl alcohol alkyl ethers; veratryl alcohol derivatives; substituted benzyl alcohol derivatives; substituted benzyl alcohol alkyl ethers; vanillin propylene glycol acetal; ethylvanillin propylene glycol acetal; ginger extract; ginger oil; gingerol; zingerone; or combinations thereof. Warming sensates are generally included in an oral care composition at a level of about 0.05% to about 2%, by weight of the oral care composition.

Abrasive polishing material can be any material that does not excessively abrade dentin. The oral care compositions of the present invention may comprise abrasive polishing material in an amount of from about 6% to about 70% or from about 10% to about 50%, by weight of the oral care composition. Typical abrasive polishing materials include silicas including gels and precipitates; aluminas; phosphates including orthophosphates, polymetaphosphates, and pyrophosphates; and mixtures thereof. Specific examples include dicalcium orthophosphate dihydrate, calcium pyrophosphate, tricalcium phosphate, calcium polymetaphosphate, insoluble sodium polymetaphosphate, rice hull silica, hydrated alumina, beta calcium pyrophosphate, calcium carbonate, and resinous abrasive materials such as particulate condensation products of urea and formaldehyde, and others such as disclosed by Cooley et al in U.S. Pat. No. 3,070,510. In certain examples, if the oral composition or particular phase comprises a polyphosphate having an average chain length of about 4 or more, calcium containing abrasives and alumina are not preferred abrasives.

Silica dental abrasives of various types are often used in oral care compositions due to their exceptional dental cleaning and polishing performance without unduly abrading tooth enamel or dentine. Silica abrasive polishing materials that may be used in the present invention, as well as other abrasives, generally have an average particle size ranging between about 0.1 to about 30 µm or from about 5 to about 15 µm. The abrasive can be precipitated silica or silica gels such as the silica xerogels described in Pader et al., U.S. Pat. No. 3,538,230 and DiGiulio, U.S. Pat. No. 3,862,307. Silica xerogels marketed under the trade name "Syloid" by the W.R. Grace & Company, Davison Chemical Division, Augusta, GA may be used. Also precipitated silica materials such as those marketed by the J. M. Huber Corporation, Edison, NJ under the trade name, "Zeodent", particularly the silica carrying the designation "Zeodent 119", may be used. The types of silica dental abrasives useful in the oral care compositions of the present invention are described in more detail in Wason, U.S. Pat. No. 4,340,583; and Rice U.S. Pat. No's 5,589,160; 5,603,920; 5,651,958; 5,658,553; and 5,716,601.

Thickening material or binders may be used to provide a desirable consistency to the oral care compositions of the present invention. For example when the oral care compositions are in the form of dentifrices, topical oral gels, mouthrinse, denture product, mouthsprays, lozenges, oral tablets or chewing gums, the amount and type of the thickening material will depend upon the form of the product. Thickening materials include carboxyvinyl polymers, carrageenan, hydroxyethyl cellulose, and water soluble salts of cellulose ethers such as sodium carboxymethylcellulose and sodium hydroxyethyl cellulose. Natural gums such as gum karaya, xanthan gum, gum arabic, and gum tragacanth can also be used. Colloidal magnesium aluminum silicate or finely divided silica can be used as part of the thickening material to further improve texture. Thickening materials can be used in an amount from about 0.1% to about 15%, by weight of the oral care composition.

Humectants keep oral care compositions from hardening upon exposure to air and certain humectants can also impart desirable sweetness of flavor to dentifrice compositions. Suitable humectants for use in the present invention include glycerin, sorbitol, polyethylene glycol, propylene glycol, xylitol, and other edible polyhydric alcohols. The oral care compositions of the present invention may comprise humectants in an amount of from about 0% to about 70% or from about 15% to about 55%, by weight of the oral care composition.

### Example: Two-Step Regimen

### First Composition

| | |
|---|---|
| Stannous Fluoride, USP | 0.454 |
| Water | 2.600 |
| Glycerin, USP (99.7%) | 58.977 |
| Zinc Lactate Dihydrate (100%) | 2.500 |
| Sodium Phosphate Tribasic Dodecahydrate | 1.100 |
| Sodium Gluconate, USP | 0.652 |
| Sodium Hydroxide (50% solution) | 0.087 |
| Xanthan Gum, NF | 0.400 |
| Sodium Carboxymethylcellulose (7M8SF)¹ | 0.200 |
| Thickening Silica (Zeodent® 165)² | 1.500 |
| Silica (Zeodent® 109)² | 12.500 |
| Silica (Zeodent® 119)² | 12.500 |
| Sodium Lauryl Sulfate (28 % solution) | 4.000 |
| Saccharin Sodium, USP (Granular) | 0.500 |
| Flavor | 1.03 |
| Colorants | 1.000 |

| | |
|---|---|
| ¹Available from Aqualon® (Wilmington, Delaware, USA) ²Available from the J. M. Huber Corporation (Edison, New Jersey, USA) | |

### Second Composition

| | |
|---|---|
| Hydrogen Peroxide (35%) | 8.700 |
| Glycerin, USP | 20.000 |
| Water | 65.400 |
| Sodium Acid Pyrophosphate | 1.000 |
| Carbopol® 956 Polymer³ (CAS# 134499-38-0) | 2.000 |
| Sodium Hydroxide (50% solution) | 0.900 |
| Saccharin Sodium, USP (Granular) | 0.500 |
| Flavor | 1.000 |
| Sucralose, USP | 0.500 |

| | |
|---|---|
| ³Available from the Goodrich Corporation (Akron, Ohio, USA) | |

### pH Test Method

First, calibrate the Thermo Scientific Orion 320 pH meter. Do this by turning on the pH meter and waiting for 30 seconds. Then take the electrode out of the storage solution, rinse the electrode with distilled water, and carefully wipe the electrode with a scientific cleaning wipe, such as a Kimwipe®. Submerse the electrode in the pH 7 buffer and press the calibrate button. Wait until the pH icon stops flashing and press the calibrate button a second time. Rinse the electrode with distilled water and carefully wipe the electrode with a scientific cleaning wipe. Then submerse the electrode into the pH 4 buffer and wait until the pH icon stops flashing and press the measure button. Rinse the electrode with distilled water and carefully wipe with a scientific cleaning wipe. Now the pH meter is calibrated and can be used to test the pH of a solution.

The pH of the liquid medication is measured using the calibrated pH meter at ambient temperature.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A kit comprising:
a. a first dispenser containing a first oral care composition comprising stannous fluoride wherein the first composition is a dentifrice and is free of linear polyphosphates wherein the first composition has a pH from 4 to 6;
b. a second dispenser containing a second oral care composition comprising from 0.1% to 6% hydrogen peroxide wherein the second composition is a gel and does not contain any fluoride salt or an abrasive wherein the second composition has a pH from 3.5 to 5.5 and wherein the pH of the first composition is higher than the pH of the second composition; and
c. instructions wherein a user is instructed to expectorate but not rinse between applying the first composition and the second composition and wherein the instructions instruct the user to use the first composition and the second composition concurrently.

2. The kit of claim 1 further comprising a manual toothbrush, preferably a manual toothbrush comprising bristles wherein the bristles are soft or extra soft.

3. The kit of claims 1 or 2 wherein the first dispenser and the second dispenser are packaged together in a secondary package comprising a transparent panel wherein at least a portion of the first dispenser or the second dispenser can be viewed through the transparent panel.

4. The kit of any of claims 1 to 3 wherein the second composition comprises from 2.5% to 3.2% hydrogen peroxide, preferably from 2.75% to 3.1% hydrogen peroxide.

5. The kit of any of claims 1 to 4 wherein the first composition has a viscosity from 5 to 40 BKUs and the second composition has a viscosity from 5 to 25 BKUs measured with a Brookfield Synchrolectic Viscometer Model RVT/2 using a T-E spindle at 2.5 revolutions per minute, at 25 °C.

6. The kit of any of claims 1 to 5 wherein the first composition has a pH from 5.25 to 6.25 and the second composition has a pH from 4.75 to 5.25 and wherein the pH of the first composition is higher than the pH of the second composition.

7. The kit of any of claims 1 to 6 wherein the first composition further comprises a first flavor and the second composition further comprises a second flavor wherein the second flavor is different and complimentary to the first flavor.

8. The kit of any of claims 1 to 7, wherein the weight ratio of first composition to second composition is from 0.5:1 to 3:1, preferably from 0.75:1 to 2:1, more preferred from 1:1 to 1.5:1.

9. The kit of any of claims 1 to 8 wherein the first and second oral care compositions are applied for 45 seconds to 90 seconds.

10. The kit of any of claims 1 to 9 wherein the second composition is applied within 45 seconds of expectorating the first composition, preferably wherein the second composition is applied within 15 seconds of expectorating the first composition.

11. The kit of any of claims 1 to 10 for use for reduction in staining, whitening, freshening breath, or as antibacterial, wherein
a. the first oral care composition is applied to an oral cavity for 30 seconds to 2 minutes;
b. the first composition is expectorated from the oral cavity;
c. the second oral care composition is applied for 30 seconds to 2 minutes; and wherein the first oral care composition and the second oral care composition are applied with a toothbrush and wherein the oral cavity is not rinsed between applying the first and second oral care compositions.

12. The kit of claim 11 wherein the kit does not significantly interfere with the taste of food and beverages after 20 minutes after expectoration.

## Patentansprüche

1. Kit, umfassend:
a. einen ersten Spender, der eine erste Mundpflegezusammensetzung enthält, umfassend Zinnfluorid, wobei die erste Zusammensetzung eine Zahncreme ist und frei von linearen Polyphosphaten ist, wobei die erste Zusammensetzung einen pH-Wert von 4 bis 6 aufweist;
b. einen zweiten Spender, der eine zweite Mundpflegezusammensetzung enthält, umfassend 0,1 % bis 6 % Wasserstoffperoxid, wobei die zweite Zusammensetzung ein Gel ist und kein Fluoridsalz oder ein Schleifmittel enthält, wobei die zweite Zusammensetzung einen pH-Wert von 3,5 bis 5,5 aufweist, und wobei der pH-Wert der ersten Zusammensetzung höher als der pH-Wert der zweiten Zusammensetzung ist; und
c. Anweisungen, wobei ein Benutzer angewiesen wird, zwischen dem Aufbringen der ersten Zusammensetzung und der zweiten Zusammensetzung auszuwerfen, aber nicht zu spülen, und wobei die Anweisungen den Benutzer anweisen, die erste Zusammensetzung und die zweite Zusammensetzung gleichzeitig zu verwenden.

2. Kit nach Anspruch 1, ferner umfassend eine manuelle Zahnbürste, vorzugsweise eine manuelle Zahnbürste, die Borsten umfasst, wobei die Borsten weich oder extra weich sind.

3. Kit nach einem der Ansprüche 1 oder 2, wobei der erste Spender und der zweite Spender zusammen in einer sekundären Packung verpackt sind, die ein transparentes Feld umfasst, wobei mindestens ein Teil des ersten Spenders oder des zweiten Spenders durch das transparente Feld hindurch betrachtet werden kann.

4. Kit nach einem der Ansprüche 1 bis 3, wobei die zweite Zusammensetzung von 2,5 % bis 3,2 % Wasserstoffperoxid, vorzugsweise von 2,75 % bis 3,1 % Wasserstoffperoxid, umfasst.

5. Kit nach einem der Ansprüche 1 bis 4, wobei die erste Zusammensetzung eine Viskosität von 5 bis 40 BKU und die zweite Zusammensetzung eine Viskosität von 5 bis 25 BKU, gemessen mit einem Brookfield Synchrolectic Viskosimeter-Modell RVT/2 unter Verwendung einer T-E Spindel bei 2,5 Umdrehungen pro Minute, bei 25 °C aufweist.

6. Kit nach einem der Ansprüche 1 bis 5, wobei die erste Zusammensetzung einen pH-Wert von 5,25 bis 6,25 aufweist und die zweite Zusammensetzung einen pH-Wert von 4,75 bis 5,25 aufweist, und wobei der pH-Wert der ersten Zusammensetzung höher als der pH-Wert der zweiten Zusammensetzung ist.

7. Kit nach einem der Ansprüche 1 bis 6, wobei die erste Zusammensetzung ferner einen ersten aromatischen Geschmacksstoff umfasst und die zweite Zusammensetzung ferner einen zweiten aromatischen Geschmacksstoff umfasst, wobei der zweite aromatische Geschmacksstoff verschieden und komplementär zum ersten aromatischen Geschmacksstoff ist.

8. Kit nach einem der Ansprüche 1 bis 7, wobei das Gewichtsverhältnis von erster Zusammensetzung zu zweiter Zusammensetzung von 0,5:1 bis 3:1, vorzugsweise von 0,75:1 bis 2:1, mehr bevorzugt von 1:1 bis 1,5:1 beträgt.

9. Kit nach einem der Ansprüche 1 bis 8, wobei die erste und die zweite Mundpflegezusammensetzung für 45 Sekunden bis 90 Sekunden aufgebracht werden.

10. Kit nach einem der Ansprüche 1 bis 9, wobei die zweite Zusammensetzung innerhalb von 45 Sekunden nach Auswerfen der ersten Zusammensetzung aufgebracht wird, vorzugsweise wobei die zweite Zusammensetzung innerhalb von 15 Sekunden nach Auswerfen der ersten Zusammensetzung aufgebracht wird.

11. Kit nach einem der Ansprüche 1 bis 10 zur Verwendung zur Reduktion von Verfärbung, zur Aufhellung, zur Auffrischung des Atems oder als antibakterielles Mittel, wobei
a. die erste Mundpflegezusammensetzung für 30 Sekunden bis 2 Minuten auf eine Mundhöhle aufgebracht wird;
b. die erste Zusammensetzung aus der Mundhöhle ausgeworfen wird;
c. die zweite Mundpflegezusammensetzung für 30 Sekunden bis 2 Minuten aufgebracht wird; und wobei die erste Mundpflegezusammensetzung und die zweite Mundpflegezusammensetzung mit einer Zahnbürste aufgebracht werden, und wobei die Mundhöhle zwischen dem Aufbringen der ersten und der zweiten Mundpflegezusammensetzung nicht gespült wird.

12. Kit nach Anspruch 11, wobei das Kit den Geschmack von Nahrungsmitteln und Getränken nach 20 Minuten nach dem Auswerfen nicht signifikant stört.

## Revendications

1. Trousse, comprenant :
a. un premier distributeur contenant une première composition de soins bucco-dentaires comprenant du fluorure stanneux dans laquelle la première composition est un dentifrice et est exempte de polyphosphates linéaires dans laquelle la première composition a un pH allant de 4 à 6 ;
b. un deuxième distributeur contenant une deuxième composition de soins bucco-dentaires comprenant de 0,1 % à 6 % de peroxyde d'hydrogène dans laquelle la deuxième composition est un gel et ne contient aucun sel fluorure ou un abrasif dans laquelle la deuxième composition a un pH allant de 3,5 à 5,5 et dans laquelle le pH de la première composition est supérieur au pH de la deuxième composition ; et
c. des instructions dans lesquelles il est indiqué à un utilisateur d'expectorer mais de ne pas rincer entre l'application de la première composition et de la deuxième composition et dans laquelle les instructions indiquent à l'utilisateur d'utiliser la première composition et la deuxième composition simultanément.

2. Trousse selon la revendication 1 comprenant en outre une brosse à dents manuelle, de préférence une brosse à dents manuelle comprenant des poils dans laquelle les poils sont doux ou extra-doux.

3. Trousse selon les revendications 1 ou 2 dans laquelle le premier distributeur et le deuxième distributeur sont conditionnés ensemble dans un conditionnement secondaire comprenant un panneau transparent dans laquelle au moins une partie du premier distributeur ou du deuxième distributeur peut être vue à travers le panneau transparent.

4. Trousse selon l'une quelconque des revendications 1 à 3 dans laquelle la deuxième composition comprend de 2,5 % à 3,2 % de peroxyde d'hydrogène, de préférence de 2,75 % à 3,1 % de peroxyde d'hydrogène.

5. Trousse selon l'une quelconque des revendications 1 à 4 dans laquelle la première composition a une viscosité allant de 5 à 40 BKU et la deuxième composition a une viscosité allant de 5 à 25 BKU mesurée avec un modèle de viscosimètre Brookfield synchroélectrique RVT/2 utilisant une broche T-E à 2,5 tours par minute, à 25 °C.

6. Trousse selon l'une quelconque des revendications 1 à 5 dans laquelle la première composition a un pH allant de 5,25 à 6,25 et la deuxième composition a un pH allant de 4,75 à 5,25 et dans laquelle le pH de la première composition est supérieur au pH de la deuxième composition.

7. Trousse selon l'une quelconque des revendications 1 à 6 dans laquelle la première composition comprend en outre un premier arôme et la deuxième composition comprend en outre un deuxième arôme dans laquelle le deuxième arôme est différent et complémentaire du premier arôme.

8. Trousse selon l'une quelconque des revendications 1 à 7, dans laquelle le rapport pondéral de la première composition à la deuxième composition est de 0,5:1 à 3:1, de préférence de 0,75:1 à 2:1, plus préférablement de 1:1 à 1,5:1.

9. Trousse selon l'une quelconque des revendications 1 à 8 dans laquelle les première et deuxième compositions de soins bucco-dentaires sont appliquées pendant 45 secondes à 90 secondes.

10. Trousse selon l'une quelconque des revendications 1 à 9 dans laquelle la deuxième composition est appliquée dans les 45 secondes suivant l'expectoration de la première composition, de préférence dans laquelle la deuxième composition est appliquée dans les 15 secondes suivant l'expectoration de la première composition.

11. Trousse selon l'une quelconque des revendications 1 à 10 utilisable pour la réduction de la coloration, le blanchiment, le rafraîchissement de l'haleine, ou comme antibactérien, dans laquelle
a. la première composition de soins bucco-dentaires est appliquée sur une cavité buccale pendant 30 secondes à 2 minutes ;
b. la première composition est expectorée de la cavité buccale ;
c. la deuxième composition de soins bucco-dentaires est appliquée pendant 30 secondes à 2 minutes ; et dans laquelle la première composition de soins bucco-dentaires et la deuxième composition de soins bucco-dentaires sont appliquées avec une brosse à dents et dans laquelle la cavité buccale n'est pas rincée entre l'application des première et deuxième compositions de soins bucco-dentaires.

12. Trousse selon la revendication 11 dans laquelle la trousse n'interfère pas de manière significative avec le goût des aliments et des boissons après 20 minutes après l'expectoration.
